# EUROPEAN PATENT SPECIFICATION

(11) **EP 3 137 147 B1**
(45) Date of publication and mention of the grant of the patent: **19.06.2019**
(21) Application number: 15786000.8
(22) Date of filing: 10.03.2015
(51) Int. Cl.: A61M 25/00, A61M 25/06, A61M 39/10

(54) **CATHETER INSTRUMENT AND CATHETER HUB THEREFORE**
KATHETERINSTRUMENT UND KATHETERNABE DAFÜR
INSTRUMENT CATHÉTER ET EMBASE DE CATHÉTER ASSOCIÉE

(30) Priority: 28.04.2014 SE 1450499
(43) Date of publication of application: 08.03.2017
(73) Proprietor: Vigmed AB, 254 66 Helsingborg (SE)
(72) Inventor: KNUTSSON, Per, S-256 55 Helsingborg (SE)
(74) Representative: Ström & Gulliksson AB
(86) International application number: PCT/SE2015/050266
(87) International publication number: WO 2015/167385

(56) References cited:
- WO-A1-99/08742
- WO-A1-2011/019316
- WO-A1-2012/039672
- WO-A1-2013/124765
- WO-A1-2013/162461
- WO-A1-2013/162461
- WO-A1-2013/187827
- WO-A2-01/68174
- US-A1- 2005 075 609
- US-A1- 2012 035 552
- US-A1- 2012 078 200
- US-A1- 2012 078 200

## Description

### Technical Field

The present invention relates to a catheter instrument, comprising a needle hub, a catheter hub, and a spring clip needle guard arranged in the catheter hub for the automatic safety shielding of a needle after its employment for introduction of a catheter tube into the vascular system of a patient. More specifically, the present invention pertains to a catheter hub for such a catheter instrument.

### Background

The clinical utilization of a pointed hollow needle mounted inside a flexible catheter tube is well known in the medical art for the introduction of a catheter. In such a medical instrument, the catheter tube is positioned tightly around the needle in such a way as to allow the needle to slide and telescope along the length of the catheter tube. Before use, the tip of the needle is protruding slightly through the opening of the catheter tube to allow facile penetration through the skin. Upon puncturing of the skin and introduction of the needle, the distal end of the catheter tube is simultaneously brought into place inside the desired target body cavity of the patient, such as the inside of a blood vessel, for example a vein. The needle has then done its duty in assisting the introduction of the catheter and is withdrawn by being pulled backwards through the catheter. Upon release of the needle, the catheter is set in its intended working mode extending over a lengthier period of time and including, for example, periodical administration or infusion of fluids or medications in liquid form, the collection of blood samples and the like.

An unprotected released needle constitutes, however, a serious health hazard due to the fact that it may be contaminated with e.g. infectious agents originating from the patient's blood or other body fluids, in combination with the needle tip's inherent ability to easily penetrate skin. Hence, the medical personnel who are handling the released needle may acquire the corresponding disease, e.g. HIV or hepatitis, if by accident contacting it with their skin. In order to circumvent or alleviate the health hazards associated with such a released needle amongst other things, there has been much effort devoted to the development of various kinds of needle tip protectors with a special focus on automatic variants of a type which may be referred to as being "foolproof".

EP 1 003 588.discloses a safety IV catheter comprising a resilient spring clip normally positioned in the catheter hub. The needle of the safety IV catheter passes through a hole in the spring clip which allows axial movement of the needle. When the needle is in the forward position, i.e. when the safety IV catheter is ready for use, the presence of the needle forces parts of the spring clip into a position where these parts locks to the inside of the catheter hub, whereby movement of the spring clip relative the catheter hub is prevented. As the needle is withdrawn to a point where the tip passes these parts, the spring clip snaps into a position in which it is blocking access to the to the tip of the needle. Simultaneously, the part of the spring clip that previously locked to the inside of the catheter hub snap out of this position, whereby movement of the spring clip relative the catheter hub may occur. As the needle is further withdrawn, means are provided, e.g. a slot or a crimp on the needle, to lock the spring clip to the needle, whereby the spring clip is ejected from the catheter hub together with, and positioned on, the needle.

For various reasons, including e.g. practical, economical and technical reasons, the above described spring clips, and similar marketed variants, are today made of metal and catheter hubs of a plastic material. Disadvantages of the combination of these materials in this application include the release of e.g. microscopic plastic chips and metallic particles by the scraping of the metal spring clip against the inside of the plastic catheter hub when the former is ejected from the latter upon withdrawal of the needle. These chips and particles may easily be flushed into the bloodstream of a patient upon normal use of the corresponding catheter, and thus represent a serious health hazard to the same. This is especially true when the spring clip needs to pass beyond a bulge or something similar within the cavity of the catheter hub, onto which the metal spring clip should be brought into retained position until being released when the needle tip passes the distal part of the metal spring clip. Another disadvantage of the spring clip of this and similar safety IV catheters is the scraping vibration generated as the needle slides through and on the spring clip as it is withdrawn. This scraping vibration, which is due to metal sliding over metal and which can be clearly heard and felt, is highly uncomfortable and worrisome to the patient, who already is in an uncomfortable and exposed situation and may be very anxious.

For these reasons attempts have been made to manufacture spring clips in materials not destroying the lumen of the catheter hub. WO2013162461 discloses a spring clip of plastic material, said spring clip interacting with the lumen of the catheter hub through a base plate thereof,

WO 2013/187827 discloses a catheter unit for use in an intravenous catheter instrument, wherein said catheter unit comprises a catheter hub and a catheter extending distally from the catheter hub according to the preamble of claim 1. However, there is a risk of the base plate interacting too much with the catheter hub lumen, since the tongues of the spring clip base plate will be in a tension state when arranged in the catheter hub. Therefore, it would be beneficial to develop a catheter instrument with less built-up tension in assembled state, to increase product reliability.

### Summary of the Invention

Accordingly, the present invention preferably seeks to mitigate, alleviate or eliminate one or more of the above-identified deficiencies in the art and disadvantages singly or in any combination and solves at least the above mentioned problems by providing a catheter unit for use in an intravenous catheter instrument, wherein said catheter unit comprises a catheter hub and a catheter extending distally from the catheter hub, said catheter having a lumen being in flow communication with an interior cavity of the catheter hub; wherein the interior cavity is provided with a cooperation groove at a proximal zone of the interior cavity.

A catheter instrument, comprising such a catheter unit, is also provided, for the same purpose.

Advantageous features of the invention are defined in the dependent claims.

### Brief Description of the Drawings

These and other aspects, features and advantages of which the invention is capable of will be apparent and elucidated from the following description of embodiments of the present invention, reference being made to the accompanying drawings, in which
Fig. 1 is a side view of a male luer fitting and a cross sectional view of a female fitting according to ISO standards;
Fig. 2 is a side view of a male luer fitting and a cross sectional view of a female fitting according to ISO standards in cooperation;
Fig. 3 is a perspective view of a catheter instrument according to the present invention, comprising a needle unit, a catheter unit, and a spring clip needle tip shielding device;
Fig. 4 is a cross-sectional view of a catheter instrument according to the present invention, comprising a needle unit, a catheter unit, and a spring clip needle tip shielding device, and a close up thereof; and
Fig. 5 is a cross-sectional view of a catheter instrument according to the present invention, comprising a needle unit, a catheter unit, and a spring clip needle tip shielding device, and a close up thereof.

### Description of embodiments

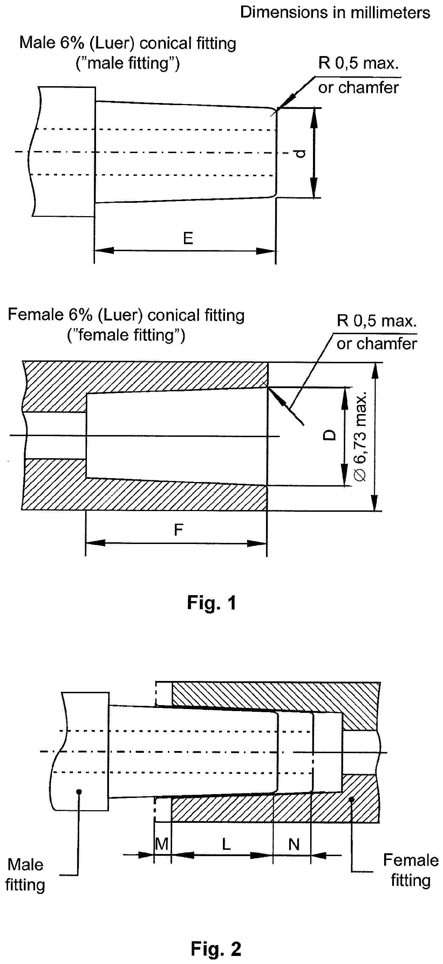

Embodiments of the present invention will be described in more detail below with reference to the accompanying Figs. 1 and 2 in order for those skilled in the art to be able to carry out the invention.

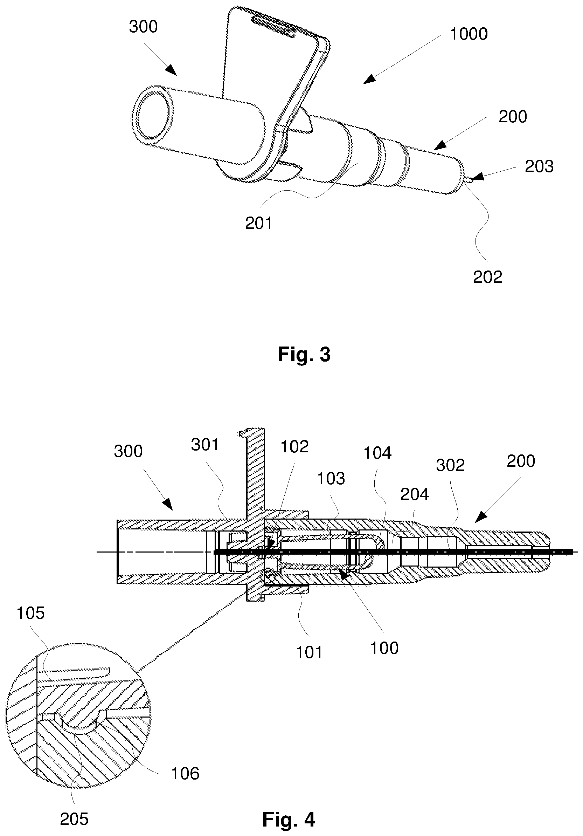

The safety IV catheter instrument 1000 includes a spring clip needle tip shielding device 100, a catheter unit 200 and a needle unit 300, in accordance with Figs. 3 to 5.

The catheter unit 200 comprises a catheter hub 201 and a catheter 202 extending distally from the catheter hub 201. The catheter 202 is hollow and tubular, and configured to house a needle stem therein. The catheter 202 is made of a suitable polymeric material. The catheter hub 201 is also made of a suitable polymeric material, such as polypropylene or polyethylene, which are cheap plastic materials with good injection molding properties. The hollow and tubular configuration of the catheter 202 provides a lumen 203 that is in flow communication with an interior cavity 204 of the catheter hub 201. The interior cavity 204 is positioned in the proximal end of the catheter hub 201, and the proximal opening into the interior cavity 204 may end in a luer fitting, such as a luer lock or luer slip, adapted to receive a tubing set, which in a known manner, administers intravenous fluid into the patient. The catheter unit 200 thus comprises a catheter hub 201 and a catheter 202 extending distally from the catheter hub 201, said catheter 202 having a lumen 203 being in flow communication with an interior cavity 204 of the catheter hub 201.

The interior cavity 204 has to follow international standards (such as ISO 594/1-1986) in respect of luer fittings. The dimensions of male and female conical fittings shall be as given in Table 1, below, and as shown in Figs. 1 and 2. The conicity is 6 degrees according to the standard.

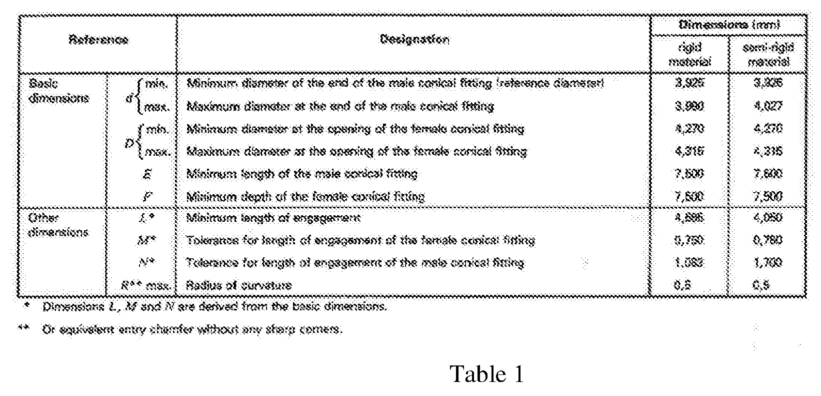

The catheter 202 is secured within an axial passageway in the distal hub section by means of a sleeve received within passageway, which engages the proximal end of the catheter 202. This passageway communicates at its proximal end with interior cavity 204, which also acts as a flash chamber, formed in catheter hub 201. The distal end of the catheter 202 may be tapered, to facilitate introduction into the vein of the patient.

The needle unit 300 of the catheter instrument 1000 comprises a needle hub 301. A needle 302 extends distally from the needle hub 301. The needle hub 301 may have an axial opening for receiving the proximal end zone of the needle 302. The needle 302 comprises a needle shaft and a needle tip, said needle tip forming the distal end point of the needle unit 300. The needle hub 301, as is conventional, may be hollow and may include a flash chamber at its proximal end. As is also conventional, the needle 302 is received within a hollow tubular catheter 202, the proximal end of which is concentrically affixed within the distal end of a catheter hub 201. At the distal end zone of the needle shaft, the needle 302 is provided with a bulge. The needle unit 300 thus comprises a needle hub 301 and a needle 302 with a needle shaft and a needle tip extending distally from the needle hub 301.

In the ready position of the catheter instrument 1000, the proximal end of the catheter hub 201 is snugly and releasably received in the distal end of the needle hub 301, such that the needle 302 extends through the cavity 204, the passageway and distally beyond the catheter hub 201 and catheter 202 so that the needle tip extends beyond a the distal end of the catheter 202. Thus, the needle hub 301 is connected to the proximal end of the catheter hub 201 and said needle shaft is arranged in the lumen 203 of the catheter 202, in a ready position of said catheter instrument 1000. The needle hub 301 may be connected to the proximal end of the catheter hub 201 and said needle shaft being arranged in the lumen 203 of the catheter 202, in a ready position of said catheter instrument 1000.

In use, the distal tip the needle 302 and the catheter 202 are inserted into a patient's vein. Thereafter, the health care practitioner manually places the catheter 202 further into the vein and then withdraws the needle by grasping and moving by hand the proximal end of the needle unit 300. The luer of the catheter hub 200, in the proximal end of the cavity 204, is then fitted with a source of the fluid that is to be administered into the patient's vein.

The spring clip needle tip shielding device 100 is arranged inside the interior cavity 204 of the catheter hub 201. The spring clip needle tip shielding device 100 comprises a base plate 101. The base plate 101 is provided with a hole 102, extending there through, i.e. from the proximal side of the base plate 101 to the distal side of the base plate. Preferably, the hole 102 is arranged centrally on the base plate 101, such that arrangement of needle 302 through said hole 102 is facilitated while the needle 302 is arranged in accordance with the ready position of the catheter instrument 1000.

A first resilient arm 103 is extending distally from an attachment point at said base plate 101. Preferably, due to manufacturing reasons, the attachment point is located at the periphery of the base plate 101. The resilient arm 103 has a resting state, from which it may be urged to yield free passage for the needle 302 through said hole 102 in an axial direction of said base plate 101 in a tension state. The resilient arm 103 is in its tension state when the catheter instrument 1000 is in its ready position. The resilient arm 103 is adapted for clamping a needle tip of a needle 302 extending through the hole 102 when the resilient arm 103 is in said resting state. For this reason, a straight imaginary line extending longitudinally through said hole 102 in the axial direction of said base plate 101 coincides with said at least one resilient arm 103 when said resilient arm 103 is in said resting state. This may be facilitated by providing the resilient arm 103 with a distal hook element 104, at the distal end of the resilient arm 103. The spring clip needle tip shielding device 100 may thus be arranged inside the interior cavity 204 of the catheter hub 201, and said needle being arranged through said hole 102 with the resilient arm 103 being urged into its tension state by said needle shaft.

The spring clip needle tip shielding device 100 is manufactured monolithically of a plastic material, with good flexibility and tension maintaining characteristics, such as polycarbonate, so as not to tear up the inner side of the catheter hub 201. The resilient arm 103 is then dimensioned such that it may be flexed into its tension state when the catheter instrument 1000 is in its ready position.

After the distal tip of the needle 302 and the catheter 202 have been inserted into a patient's vein, the needle unit 300 is displaced proximally in relation to the catheter unit 200 and the spring clip needle tip shielding device 100. The spring clip needle tip shielding device 100 is retained in the catheter hub 201 of the catheter unit 200 through interaction between the base plate 102, in accordance with above. When the needle unit 300 is displaced proximally in relation to the catheter unit 200 and the spring clip needle tip shielding device 100, also the needle 302 is displaced proximally in relation to these two. Once the needle tip passes proximally beyond the distal end of the resilient arm 103, such as the hook element 104, the distal end of the resilient arm 103 snaps in front of the needle tip. The bulge on the needle shaft then hits the base plate 102, since the bulge has been dimensioned with a somewhat larger diameter than the through hole of the base plate 102. Also, the bulge has been positioned on the needle shaft at a distance from the needle tip largely corresponding to the distance between the base plate 102 and the distal end of the resilient arm 103, such that the spring clip needle tip shielding device 100 may be secured at the distal end of the needle 302 once the needle tip has been displaced proximally beyond the distal end, such as the hook element 104, of the spring clip needle tip shielding device 100. In this position, the spring clip needle tip shielding device 100 is released from the catheter unit 200 through overcoming the frictional force between the base plate 102 and the interior wall of the catheter hub 201, in accordance with above.

The spring clip needle tip shielding device 100 may be provided with more than one resilient arm 103. This additional resilient arm may also traverse the central axis of the catheter unit 200, and may thus also be provided with a through hole or a cut-out for letting the needle 301 pass there through. An additional resilient arm may further stabilize the positioning of the spring clip needle tip shielding device 100 on the needle shaft 300. Also the second resilient arm may be provided with a distal hook element for central displacement once the needle tip have passed proximally beyond the distal end of the first and second resilient arms 103.

The spring clip shielding device 100 is held in place in the catheter hub 201 through friction between the base plate 102 and the interior wall of the catheter hub 201. The base plate 102 of the spring clip needle tip shielding device 100 is provided with tongues 105. The tongues 105 may in turn be provided with cooperation knobs 106. The cooperation knobs 106 extend laterally outwards from the base plate 102. In this way, the cooperation knobs 106 may flex somewhat to facilitate cooperation between the base plate 102, and thus the spring clip shielding device 100, and the catheter hub 200, and also compensate for discrepancies in shape between the base plate 102 and the lumen of the catheter hub.

In a luer fitting, according to the standard indicated above in Table 1 and Fig. 1, there is a tolerance M. This tolerance M may be used for further improving engagement between the base plate 102 of the spring clip shielding device 100 and the interior cavity 204 of the catheter hub 201. For this reason, the interior cavity 204 is provided with a cooperation groove 205. The cooperation groove 205 is thus positioned within a distance from the proximal end, i.e. a proximal zone, of the catheter hub 201 of 0.75 mm (M, in Figs. 1 and 2), such as 0.5 mm, such as 0.45+/-0.05 mm. The cooperation groove 205 extends radially in the interior cavity 204, i.e. on the inside of the catheter hub 201. The depth of the cooperation groove is 0.05 to 0.1 mm, such as 0.06 to 0.08 mm. When receiving the spring clip shielding device 100 in the interior cavity 204 of the catheter hub 201, the cooperation knobs 106 on the tongues 105 will snap into cooperation with the catheter hub 200 through first being urged centrally when being pushed distally into the catheter hub, to thereafter relax to snap into the cooperation groove 205, as disclosed in Fig. 4. In this way, the knobs 106, and thereby tongues 105 and spring clip shielding device 100, will be in a more relaxed state in mounted position, whereby the risk of plastic-to-plastic migration is relieved - hence improving long-term storing of the catheter instrument 1000.

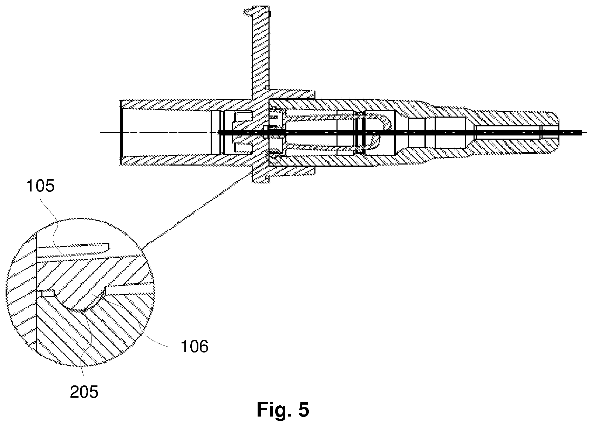

It is also possible to dimension the relationship between the groove 205, tongues 105 and the knobs 106, such that the tongues 105 and knobs 106 are in a more relaxed state, but still in physical contact with the groove 205, as disclosed in Fig. 5.

Although the present invention has been described above with reference to specific embodiments, it is not intended to be limited to the specific form set forth herein. Rather, the invention is limited only by the accompanying claims and, other embodiments than the specific above are equally possible within the scope of these appended claims.

In the claims, the term "comprises/comprising" does not exclude the presence of other elements or steps. Furthermore, although individually listed, a plurality of means, elements or method steps may be implemented by e.g. a single unit or processor. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality.

## Claims

1. A catheter unit (200) for use in an intravenous catheter instrument (1000), wherein said catheter unit (200) comprises a catheter hub (201) and a catheter (202) extending distally from the catheter hub (201), said catheter (202) having a lumen (203) being in flow communication with an interior cavity (204) of the catheter hub (201);
**characterized in that**
the interior cavity (204) is provided with a cooperation groove (205) at a proximal zone of the interior cavity (204);
wherein the proximal zone extends distally 0.75 mm from a proximal end of the interior cavity (204).

2. The catheter unit (200) according to claim 1, wherein the proximal zone extends distally 0.45+/-0.05 mm from a proximal end of the interior cavity (204).

3. The catheter unit (200) according to any of the preceding claims, wherein the depth of the cooperation groove (205) is 0.05 to 0.1 mm.

4. The catheter unit (200) according to any of the preceding claims, wherein the cooperation groove (205) extends radially in form of a ring on the inside of the catheter hub (201).

5. A catheter instrument (1000) comprising a catheter unit (200) according to any of claims 1 to 4, a spring clip needle tip shielding device (100) and a needle unit (300);
wherein the spring clip needle tip shielding device (100) comprises: a base plate (101) with a hole (102) extending there through; at least one resilient arm (103) extending at an attachment point at said base plate (101); wherein said at least one resilient arm (103) has a resting state, from which it may be urged to yield free passage through said hole (102) in an axial direction of said base plate (101) in a tension state, said at least one resilient arm (103) being adapted for clamping a needle tip of a needle (301) extending through said hole (102) when said resilient arm (103) is in said resting state;
wherein said needle unit (300) comprises a needle hub (301) and a needle (302) with a needle shaft and a needle tip extending distally from the needle hub (301);
said needle hub (301) being connected to the proximal end of the catheter hub (201) and said needle shaft being arranged in the lumen (203) of the catheter (202), in a ready position of said catheter instrument (1000), and
said spring clip needle tip shielding device (100) being arranged inside the interior cavity (204) of the catheter hub (201), and said needle being arranged through said hole (102) with the resilient arm (103) being urged into its tension state by said needle shaft.

6. The catheter instrument (1000) according to claim 5, wherein tongues (105) with knobs (106) on said base plate (102) cooperate releasably with the cooperation groove (205).

## Patentansprüche

1. Kathetereinheit (200) zur Verwendung in einem intravenösen Katheterinstrument (1000), wobei besagte Kathetereinheit (200) einen Katheterhub (201) und einen Katheter (202) umfasst, der sich distal von dem Katheterhub (201) erstreckt, wobei besagter Katheter (202) ein Lumen (203) aufweist, das in Fließverbindung mit einem inneren Hohlraum (204) des Katheterhubs (201) steht;
**dadurch gekennzeichnet, dass**
der innere Hohlraum (204) mit einer Kooperationsnut (205) in einer proximalen Zone des inneren Hohlraums (204) versehen ist;
wobei sich die proximale Zone distal 0,75 mm von einem proximalen Ende des inneren Hohlraums (204) erstreckt.

2. Kathetereinheit (200) nach Anspruch 1, wobei sich die proximale Zone distal 0,45+/-0,05 mm von einem proximalen Ende des inneren Hohlraums (204) erstreckt.

3. Kathetereinheit (200) nach einem der vorangehenden Ansprüche, wobei die Tiefe der Kooperationsnut (205) 0,05 bis 0,1 mm beträgt.

4. Kathetereinheit (200) nach einem der vorangehenden Ansprüche, wobei sich die Kooperationsnut (205) radial in Form eines Ringes auf der Innenseite des Katheterhubs (201) erstreckt.

5. Katheterinstrument (1000), umfassend eine Kathetereinheit (200) nach einem der Ansprüche 1 bis 4, eine Federklammer-Nadelspitzenabschirmvorrichtung (100) und eine Nadeleinheit (300);
wobei die Federklammer-Nadelspitzenabschirmvorrichtung (100) umfasst: eine Grundplatte (101) mit einem Loch (102), das sich durch sie hindurch erstreckt; mindestens einen elastischen Arm (103), der sich von einem Befestigungspunkt an besagter Grundplatte (101) aus erstreckt; wobei der besagte mindestens eine elastische Arm (103) einen Ruhezustand aufweist, aus dem er veranlasst werden kann, einen freien Durchgang durch das Loch (102) in axialer Richtung der Grundplatte (101) in einem Spannungszustand zu ermöglichen, wobei der mindestens eine elastische Arm (103) zum Klemmen einer Nadelspitze einer Nadel (301) geeignet ist, die sich durch das Loch (102) erstreckt, wenn sich der elastische Arm (103) in besagtem Ruhezustand befindet;
wobei die besagte Nadeleinheit (300) einen Nadelhub (301) und eine Nadel (302) mit einem Nadelschaft und einer Nadelspitze umfasst, die sich distal von dem Nadelhub (301) erstreckt;
wobei besagter Nadelhub (301) mit dem proximalen Ende des Katheterhubs (201) verbunden ist und besagter Nadelschaft in dem Lumen (203) des Katheters (202) in einer fertigen Position des Katheterinstruments (1000) angeordnet ist, und
besagte Federklammer-Nadelspitzenabschirmvorrichtung (100) im Inneren des inneren Hohlraums (204) des Katheterhubs (201) angeordnet ist, und besagte Nadel durch das Loch (102) hindurch mit dem elastische Arm (103) durch den Nadelschaft in seinen Spannungszustand gedrückt, angeordnet ist.

6. Katheterinstrument (1000) nach Anspruch 5, wobei Zungen (105) mit Noppen (106) auf besagter Grundplatte (102) lösbar mit der Kooperationsnut (205) zusammenwirken.

## Revendications

1. Unité cathéter (200) destinée à être utilisée dans un instrument (1000) formant cathéter intraveineux, dans laquelle ladite unité cathéter (200) comprend une embase (201) de cathéter et un cathéter (202) s'étendant de manière distale de ladite embase (201) de cathéter, ledit cathéter (202) comportant une lumière (203) en communication d'écoulement avec une cavité intérieure (204) de l'embase (201) de cathéter ;
**caractérisée en ce que**
la cavité intérieure (204) est pourvue d'une rainure de coopération (205) au niveau d'une zone proximale de la cavité intérieure (204) ;
dans laquelle la zone proximale s'étend de manière distale de 0,75 mm d'une extrémité proximale de la cavité intérieure (204).

2. Unité cathéter (200) selon la revendication 1, dans laquelle la zone proximale s'étend de manière distale de 0,45 ±0,05 mm d'une extrémité proximale de la cavité intérieure (204).

3. Unité cathéter (200) selon l'une quelconque des revendications précédentes, dans laquelle la profondeur de la rainure de coopération (205) va de 0,05 à 0,1 mm.

4. Unité cathéter (200) selon l'une quelconque des revendications précédentes, dans laquelle la rainure de coopération (205) s'étend radialement sous la forme d'un anneau du côté intérieur de l'embase (201) de cathéter.

5. Instrument (1000) formant cathéter comprenant une unité cathéter (200) selon l'une quelconque des revendications 1 à 4, un dispositif de protection à pince à ressort (100) de pointe d'aiguille et une unité aiguille (300) ;
dans lequel le dispositif de protection à pince à ressort (100) de pointe d'aiguille comprend : une plaque de base (101) dotée d'un trou (102) la traversant ; au moins un bras élastique (103) s'étendant au niveau d'un point de fixation au niveau de ladite plaque de base (101) ; dans lequel ledit au moins un bras élastique (103) a un état au repos, à partir duquel il peut être poussé pour établir un passage libre à travers ledit trou (102) dans une direction axiale de ladite plaque de base (100) dans un état de traction, ledit au moins un bras élastique (103) étant conçu pour pincer une pointe d'aiguille d'une aiguille (301) s'étendant à travers ledit trou (102) lorsque ledit bras élastique (103) est dans ledit état au repos ;
dans lequel ladite unité aiguille (300) comprend un raccord (301) d'aiguille et une aiguille (302) dotée d'une tige d'aiguille et d'une pointe d'aiguille s'étendant de manière distale du raccord (301) d'aiguille ;
ledit raccord (301) d'aiguille étant raccordé à l'extrémité proximale de l'embase (201) de cathéter et ladite tige d'aiguille étant disposée dans la lumière (203) du cathéter (202), dans une position prêt à l'utilisation dudit instrument (1000) formant cathéter, et
ledit dispositif de protection à pince à ressort (100) de pointe d'aiguille étant disposé à l'intérieur de la cavité intérieure (204) de l'embase (201) de cathéter, et ladite aiguille étant disposée à travers ledit trou (102), le bras élastique (103) étant poussé dans son état de traction par ladite tige d'aiguille.

6. Instrument (1000) formant cathéter selon la revendication 5, dans lequel des languettes (105) dotées de boutons (106) situés sur la plaque de base (102) coopèrent de manière libérable avec la rainure de coopération (205).
